## Europäisches Patentamt

## European Patent Office

## Office européen des brevets

(19)

(11) **EP 0 925 382 B1**

(12) **EUROPÄISCHE PATENTSCHRIFT**

(45) Veröffentlichungstag und Bekanntmachung des Hinweises auf die Patenterteilung:
**20.11.2002  Patentblatt 2002/47**

(21) Anmeldenummer: **97943774.6**

(22) Anmeldetag: **12.09.1997**

(51) Int Cl.[7]: **C23C 14/32**, C23C 14/06, C23C 26/02, C23C 28/00

(86) Internationale Anmeldenummer:
**PCT/DE97/02071**

(87) Internationale Veröffentlichungsnummer:
**WO 98/011272 (19.03.1998 Gazette 1998/11)**

(54) **VERSCHLEISSBESTÄNDIGER, MECHANISCH HOCHBELASTBARER UND REIBUNGSARMER RANDSCHICHTAUFBAU AUS EINEM SUBSTRAT AUS TITAN ODER SEINEN LEGIERUNGEN SOWIE VERFAHREN ZU SEINER HERSTELLUNG**

WEAR-RESISTANT EDGE LAYER STRUCTURE ON A SUBSTRATE MADE OF TITANIUM OR ITS ALLOYS WHICH CAN BE SUBJECTED TO A HIGH MECHANICAL LOAD AND HAS A LOW COEFFICIENT OF FRICTION, AND METHOD OF PRODUCING THE SAME

STRUCTURE DE COUCHE MARGINALE SUR UN SUBSTRAT DE TITANE OU DE SES ALLIAGES, RESISTANT A L'USURE, POUVANT ETRE FORTEMENT SOLLICITEE SUR LE PLAN MECANIQUE ET A FAIBLE COEFFICIENT DE FROTTEMENT, ET PROCEDE PERMETTANT DE LA REALISER

(84) Benannte Vertragsstaaten:
**CH DE FR GB IT LI NL SE**

(30) Priorität: **13.09.1996  DE 19637450**

(43) Veröffentlichungstag der Anmeldung:
**30.06.1999  Patentblatt 1999/26**

(73) Patentinhaber: **FRAUNHOFER-GESELLSCHAFT ZUR FÖRDERUNG DER ANGEWANDTEN FORSCHUNG E.V. 80636 München (DE)**

(72) Erfinder:
 • **BRENNER, Berndt**
   **D-01474 Pappritz (DE)**
 • **BONSS, Steffen**
   **D-98544 Zella-Mehlis (DE)**
 • **SCHEIBE, Hans-Joachim**
   **D-01309 Dresden (DE)**
 • **ZIEGELE, Holger**
   **D-73547 Lorch (DE)**

(56) Entgegenhaltungen:
| | |
|---|---|
| EP-A- 0 105 835 | EP-A- 0 242 100 |
| EP-A- 0 246 828 | EP-A- 0 322 812 |
| EP-A- 0 491 075 | EP-A- 0 592 309 |
| WO-A-90/14447 | WO-A-95/26169 |
| US-A- 5 368 939 | |

 • **KOLITSCH A ET AL: "MODIFICATION OF LASER-ARC DLC LAYERS BY ION BEAMS" PROCEEDINGS. INTERNATIONAL SYMPOSIUM TRENDS NEW APPLICATIONS ON THIN FILMS, 1.Januar 1993, Seiten 353-356, XP000570625**

**Beschreibung**

Die Erfindung bezieht sich auf die Randschichtveredlung von Funktionsbauteilen. Objekte, bei denen ihre Anwendung möglich und zweckmäßig ist, sind alle gleitverschleißbeanspruchten Funktionsbauteile aus Titan oder seinen Legierungen, die bei Einsatztemperaturen unter 500 °C belastet werden, einer hohen Flächenpressung unterworfen sind und einen möglichst geringen Reibungskoeffizienten aufweisen müssen. Besonders vorteilhaft ist die Erfindung zum Schutz von Humanimplantaten, insbesondere mit oszillierenden Bewegungsabläufen sowie gleitverschleißbeanspruchten Bauteilen aus dem Luft- und Raumfahrtsektor einsetzbar.

Titan ist ein hervorragender Konstruktionswerkstoff, dessen hohe spezifische Festigkeit, chemische Beständigkeit und Biokompatibilität ihn für verschiedene Sonderanwendungen prädestiniert. Einer größeren Einsatzbreite steht jedoch vielfach seine geringe Beständigkeit gegenüber Gleitverschleiß und sein hoher Reibungskoeffizient entgegen.

Es ist bekannt, sehr verschteißbeständige Randschichten auf Titan durch Lasergaslegieren zu erzeugen (vgl. z. B. H. W. Bergmann: »Thermochemische Behandlung von Titan und Titanlegierungen durch Laserumschmelzen und Gaslegieren«, Zeitschrift für Werkstofftechnik 16 (1985), S. 392 405).

Darüberhinaus ist bekannt, das Lasergaslegieren zum Schutz von Gelenkendoprothesen zu verwenden (PS DE 3 917 211). Dazu wird das Bauteil durch den Laserstrahl bis auf eine Tiefe von 0,1 bis 0,7 mm aufgeschmolzen und gleichzeitig Stickstoff in die Schmelze eingeblasen. Wegen der hohen Affinität des Titans zu reaktiven Gasen bildet sich sofort Titannitrid, das sich in Form von Nadeln aus der Schmelze ausscheidet. Nach der Erstarrung besteht die Randschicht aus einer metallischen Matrix aus Titan mit einem gegenüber dem Ausgangszustand veränderten α/β-Anteil sowie sehr dicht eingelagerten teilweise recht groben Titannitridnadeln. Die Härte der Randschicht beträgt üblicherweise bis 1000 HV.

Der Mangel solcher Schichten besteht jedoch darin, daß sie einen großen Reibungskoeffizient aufweisen und darüberhinaus einen starken Abrieb bei den meisten verwendbaren Gegenkörpern hinterlassen. Die Ursache dieses Mangels besteht darin, daß die sehr harten Titannitridnadeln nach dem Einlaufverschleiß aus der Oberfläche hervorragen. Dadurch wird die lokale Beanspruchung des Tribosystems bis zur Furchung des Gegenkörpers erhöht und gleichzeitig mikroskopische Verhakungen der Titannitrid-nadeln mit dem Gegenkörper bewirkt, die den Reibungskoeffizient erhöhen.

Ein weiterer Mangel dieser Schichten tritt unter Belastung in einer sauerstoffhaltigen Atmosphäre und bevorzugt unter höheren Temperaturen auf und äußert sich darin, daß es besonders unter Mangelschmierbedingungen zu einem katastrophalen Versagen der Reibpaarung kommen kann. Die Ursache hierfür besteht darin, daß die metallische Matrix zwischen den TiN-Nadeln eine hohe Affinität zum Sauerstoff hat.

Um die negativen Auswirkungen der TiN-Nadeln, insbesondere für den Anwendungsbereich der Humanimplantate, umgehen zu können, ist ein Verfahren zum Gasnitrieren (PS US 5,326,362) bekannt geworden, bei dem molekularer Stickstoff bei einer Prozeßtemperatur von 400 °C bis 704,4 °C in die oberflächennahen Bereiche eindiffundiert und durch eine Lösungshärtung eine verschleißfeste Randschicht bildet. Dazu wird das Bauteil in einem Vakuumofen plaziert, auf ein Druck von $1 \cdot 10^{-6}$ Torr evakuiert, anschließend mit 1 at Stickstoff aufgefüllt, auf 537,7 °C aufgeheizt, der Stickstoffdruck auf 2 at erhöht und mehrere Stunden bei 593,3 °C nitriert. Nach Abschluß der Behandlung besteht die Randschicht aus einer 0,2 μm dicken Verbindungsschicht aus Titannitriden, Titancarbonitriden, Titanoxiden und - carbo-oxiden und einer einige μm dikken Diffusionsschicht. Die sich in der Verbindungsschicht befindlichen Titannitride sind bedeutend feindisperser als beim Lasergaslegieren. Da die Verbindungsschicht eine geschlossene Schicht auf der Oberfläche bildet, wird damit gleichzeitig die Belastungsfähigeit der Schicht unter sauerstoffhaltiger Atmosphäre und erhöhten Temperaturen verstärkt.

Die Mängel dieses Verfahrens bestehen jedoch darin, daß der Gleitreibungskoeffizient nicht ausreichend herabgesetzt wird und daß die Verschleißbeständigkeit bei hohen Kontaktdrücken nicht ausreichend ist. Die Ursache dieser Mängel resultiert daraus, daß einerseits die Verbindungsschicht weiterhin aus sehr harten und nicht völlig ebenen Titannitrid-Nadeln besteht, die den Gegenkörper furchen und andererseits die darunterliegende Diffusionsschicht zu dünn ist, um einer hohen lokalen Belastung ausreichend lange widerstehen zu können. Letzteres resultiert in erster Linie daraus, daß bei einer Hertz'schen Pressung mit den in der Praxis auftretenden Kontaktflächen das Spannungsmaximum unter der Schicht liegt. Im weichen Grundmaterial kann es deshalb zu plastischen Deformationen kommen, die zu einem Abheben der spröden Verbindungsschicht führen.

Ziel der Erfindung ist es, einen vor Gleitverschleiß deutlich beständigeren, biokompatiblen Randschichtaufbau mit sehr geringen Gleitreibungskoeffizienten für Titan und seine Legierungen anzugeben und ein Verfahren zu seiner Herstellung vorzuschlagen.

Der Erfindung liegt die Aufgabe zu Grunde, einen Randschichtaufbau anzugeben, der unter Nutzung der hohen Verschleißbeständigkeit des Titannitrids eine um mindestens eine Größenordnung höhere Einhärtetiefe aufweist und der direkt in der Oberfläche keine Titannitrid-Nadeln enthält.

Erfindungsgemäß wird diese Aufgabe mit einem verschleißbeständigen, mechanisch hochbelastbaren und reibungsarmen Randschichtaufbau auf ein Substrat aus Titan oder seinen Legierungen, bestehend aus einer lasergaslegierten Schicht mit ausgeschiedenen Titannitridnadeln, wie in den Anspüchen 1 oder 2 ausgeführt, gelöst.

Anspruch 2 stellt eine vorteilhafte Ausgestaltung der Zwischenschicht dar, bei der eine besonders gute Haftung der hart-amorphen Kohlenstoffschicht erreicht wird.

Außerdem wird die Aufgabe durch ein Verfahren zur Erzeugung eines Randschichtaufbaues mit niedrigen Reibungskoeffizienten und einer sehr hohen Lasttragfähigkeit gelöst. Erfindungsgemäß wird dabei wie in Anspruch 3 angegeben, vorgegangen.

Die Erfindung ist am nachfolgenden Ausführungsbeispiel näher erläutert.

In den dazugehörigen Zeichnungen sind der erfindungsmäßige Schichtaufbau (Zeichnung 1) sowie die Verbesserung des Verschleißverhaltens (Zeichnung 2 a) und des Reibungsverhaltens (Zeichnung 2 b) dargestellt.

**Beispiel 1:**

Ein Bauteil aus der Legierung Ti6Al4V mit den Abmaßen 10 x 40 x 60 mm$^3$ soll an einer Flachseite mit einem sehr verschleißbeständigen und reibungsarmen Schichtaufbau versehen werden.

Dazu wird das geschliffene und mit einem Lösungsmittel von Fettrückständen gereinigte Bauteil auf dem Bearbeitungstisch einer CNC-Maschine positioniert. Über dem Bauteil befindet sich eine spezielle Schutzgesglocke, in die ein in einer Gasmischstation eingestelltes $N_2$-Ar-Gemisch bei Luftdruck im Verhältnis $N_2$ : Ar = 60 : 40 bzw. $N_2$: Ar = 70 : 30 eingeblasen wird. Die Schutzglocke ist so konstruiert, daß sie in drei Koordinatenrichtungen frei beweglich ist und schon nach einer Spülzeit von 90 s einen Sauerstoffrestgehalt $\leq$ 3 ppm garantiert. Die Laserleistung beträgt 5 kW, der Strahl hat auf dem Bauteil einen Durchmesser von 3,4 mm, als Vorschubgeschwindigkeit wird 8 mm/min gewählt. Der Spurabstand beträgt 0,75 mm bei einer sich ergebenden Spurbreite von 4,5 mm. Daraus ergibt sich ein Überlappungsgrad von 83,3 %. Nach Erreichen des Sauerstoffpartialdruckes < 5 ppm wird der Prozeß des Gaslegierens durch den Start des CNC-Programms und die Laserstrahlfreigabe gestartet. Die so erzeugte Schicht ist 1,2 mm tief, besteht aus Titannitridnadeln, die in einer Titanmatrix eingebettet sind.

Nach Erkalten des Bauteiles wird dessen Oberfläche mit einer diamantgebundenen Schleifscheibe der Körnung 125 μm geschliffen, bis eine gleichmäßige Oberfläche mit einer Rauhigkeit $\leq$ 0,62 μm erreicht ist. Danach erfolgt das Schleifen mit SiC-Papier P800 bis P1200, bis eine Rauhheit von $\leq$ 0,12 μm erreicht ist.

Anschließend wird die Oberfläche des Bauteiles in einer Vakuumkammer durch Ionenbeschuß mit Argon-Ionen gereinigt. In der gleichen Kammer befindet sich eine Anordnung zur Durchführung des lasergesteuerten, gepulsten Vakuumbogen (Laser-Arc) - Prozesses zur Erzeugung der hart-amorphen Kohlenstoffschichten. Die Anordnung besteht aus einem gepulsten Vakuumbogen, dem als Kathode geschalteten Targetmaterial sowie einem gütegeschalteten Nd-Yag-Laser mit einer Leistungsdichte > 5 • $10^8$ W/cm$^2$. Das Bauteil ist in einem Substrathalter befestigt.

Die walzenförmige Kathode ist zweigeteilt und besteht aus einer Titan- und einer Graphitwalze.

Zwischen Kathode und Anode wird eine Spannung angelegt, die noch nicht zur Zündung einer Bogenentladung ausreicht. Der Laser wird auf die Titanwalze fokussiert und angeschaltet. Seine Pulse erzeugen Plasmawolken, die jeweils eine kurze Bogenentladung zünden. Auf dem Substrat werden Ti-Ionen und -Atome abgeschieden. Nach Erreichen der erwünschten Schichtdicke der Zwischenschicht wird auf die Graphit-Kathode umgeschaltet und die hart-amorphe Kohlenstoffschicht auf dem Bauteil abgeschieden. Die abgeschiedene Schicht ist 400 nm dick und besteht aus einem hart-amorphen Kohlenstofffilm.

Zur Bestimmung des Verschleißwiderstandes werden die Bauteile auf einem Kugel-Scheibe-Tribometer getestet, wobei die Kugel aus-Hartmetall besteht.

**Patentansprüche**

1. Verschleißbeständiger, hochbelastbarer und reibungsarmer Randschicht-aufbau auf einem Substrat des Titan und dessen Legierungen aus einer lasergaslegierten Schicht mit ausgeschiedenen Titannitrid-Nadeln, **dadurch gekennzeichnet, daß** die Randschicht aus

   • einer 200 ... 400 nm dicken hart-amorphen Kohlenstoffschicht 4
   • einer 50 ... 200 nm dicken Zwischenschicht 3
   • und einer 0,3 ... 2,0 mm dicken lasergaslegierten Schicht 2 besteht

   und die gaslegierte Schicht eine Härte zwischen 600 HV0,1und 1400 HV0,1 aufweist.

2. Randschichtaufbau nach Anspruch 1, **dadurch gekennzeichnet, daß** die Zwischenschicht aus Titan besteht.

3. Verfahren zur Herstellung eines verschleißbeständigen, hochbelastbaren und reibungsarmen Randschichtaufbaues auf einem Substrat aus Titan und dessen Legierungen nach Anspruch 1 und 2, **dadurch gekennzeichnet, daß**

   a. die zu schützende Bauteiloberfläche spurweise mit einem Hochleistungslaser aufgeschmolzen wird,
   b. die Leistungsdichte p des Laserstrahles 1 •

$10^4$ W/cm$^2 \leq p \leq 2 \cdot 10^5$ W/cm$^2$ beträgt,

c. das Aufschmelzen bei einem Sauerstoffpartialdruck < 5 ppm passiert,

d. die reaktive Atmosphäre aus N$_2$ und Ar besteht, wobei ein relativer Stickstoffgehalt zwischen 40 % und 80 % gewählt wird,

e. ein Überlappungsgrad Ü;

$$\ddot{U} = \frac{a\text{-}c}{a}$$

ausgedrückt im Verhältnis (Spurbreite a - Spurabstand c): Spurbreite a von $0{,}5 \leq \ddot{U} \leq 0{,}9$ gewählt wird,

f. das Bauteil anschließend bis mindestens zu einer Oberflächenrauhigkeit $\leq 0{,}2$ µm geschliffen wird,

g. in einer Hochvakuumeinrichtung durch Ionenbeschuß gereinigt wird,

h. anschließend mit dem lasergesteuerten, gepulsten Vakuumbogen (Laser-Arc) - Verfahren die Zwischenschicht aufgebracht wird, Arc)-Verfahren die Zwischenschicht aufgebracht wird,

i. und abschließend in der gleichen Anordnung mit dem lasergesteuerten, gepulsten Vakuumbogen(Laser-Arc)-Verfahren eine hart-amorphe Kohlenstoffschicht auf dem Bauteil abgeschieden wird.

## Claims

1. A wear-resistant edge layer structure on a substrate of titanium and its alloys, which can be highly loaded and has low friction, consisting of a laser gas alloyed layer with precipitated titanium nitride needles, **characterized in that** the edge layer consists of

   • a hard, amorphous carbon layer 4, 200 - 400 nm thick,
   • an intermediate layer 3, 50 - 200 nm thick,
   • and a laser gas alloyed layer 2, 0.3 - 2.0 mm thick

   and the gas alloyed layer has a hardness between 600 HV0.1 and 1400 HV0.1.

2. An edge layer structure according to claim 1, **characterized in that** the intermediate layer consists of titanium.

3. A method of making a wear-resistant edge layer structure on a substrate of titanium and its alloys, which can be highly loaded and has low friction, according to claim 1 or 2, **characterized in that**

   a. the component surface to be protected is melted along tracks by a high power laser,

b. the power density p of the laser beam amounts to $1 \cdot 10^4$ W/cm$^2 \leq p \leq 2 \cdot 10^5$ W/cm$^2$,

c. the melting takes place in an oxygen partial pressure < 5 ppm,

d. the reactive atmosphere consists of N$_2$ and Ar, where a relative content of nitrogen is selected between 40% and 80%,

e. a degree of overlap U; U = (**a** - **c**)/**a** expressed as the ratio of (track width **a** - track pitch **c**) to track width **a** is selected with $0.5 \leq U \leq 0.9$,

f. the component is then ground down to a surface roughness at least $\leq 0.2$ µm,

g. it is cleaned by ion bombardment in a high vacuum device,

h. the intermediate layer is then applied by the laser controlled, pulsed vacuum arc (laser arc) method,

i. and finally a hard, amorphous carbon layer is deposited on the component in the same arrangement by the laser controlled, pulsed vacuum arc (laser arc) method.

## Revendications

1. Structure de couche marginale sur un substrat de titane et d'alliages de titane, pouvant être fortement sollicité et résistant à l'usure, composée d'une couche alliée au gaz par laser et à un dépôt d'aiguilles de nitrure de titane,
   **caractérisée en ce que**
   la couche est composée :

   - d'une couche (4) de carbone amorphe dur d'une épaisseur de 200 à 400 nm,
   - d'une couche intermédiaire (3) d'une épaisseur de 50 à 200 nm,
   - d'une couche (4) alliée au gaz par laser, d'une épaisseur de 0,3 à 2,0 mm, présentant une dureté comprise entre 600 HVO, 1 et 1400 HVO,1.

2. Structure de couche selon la revendication 1,
   **caractérisée en ce que**
   la couche intermédiaire est en titane.

3. Procédé de fabrication sur un substrat de titane et d'alliages de titane, d'une structure de couche marginale résistant à l'usure, pouvant être fortement sollicitée et à faible coefficient de frottement,
   **caractérisé en ce que**

   a. la surface du composant à protéger est fondue selon des pistes, au moyen d'un laser à haute puissance,

   b. la densité de puissance p du faisceau laser est comprise entre $1 . 10^4$ W/cm$^2 \leq p \leq 2 . 10^5$ W/cm$^2$,

c. la fusion s'effectue à une pression partielle d'oxygène < 5 ppm,

d. l'atmosphère réactive est composée d'azote et d'argon, la teneur relative en azote étant choisie entre 40 % et 80 %,

e. on choisit un taux de superposition Ü entre 0,5 et 0,9 avec :

$$\ddot{U} = \frac{a - c}{a}$$

a étant la largeur de la piste, et c l'entraxe des pistes,

f. le composant est ensuite rectifié jusqu'à obtention d'une rugosité superficielle < à 0,2 $\mu$m,

g. le composant est nettoyé dans un dispositif à vide, par bombardement ionique,

h. la couche intermédiaire est réalisée par le procédé à arc pulsé sous vide, commandé par laser (arc laser),

i. et enfin, dans le même système et par le procédé à arc pulsé sous vide, commandé par laser (arc laser) une couche de carbone amorphe dur est déposée sur le composant.

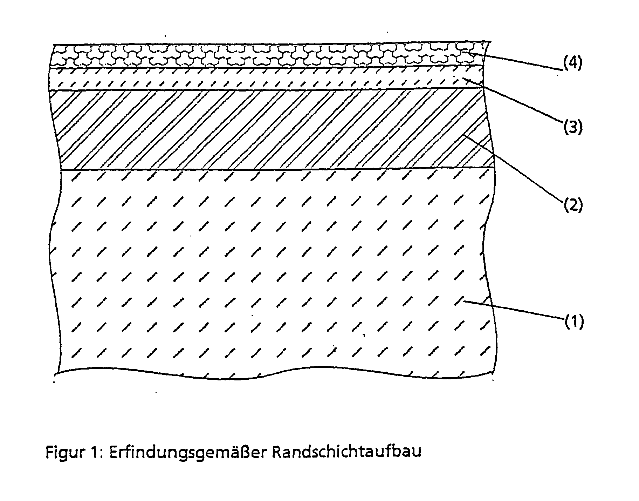

Figur 1: Erfindungsgemäßer Randschichtaufbau

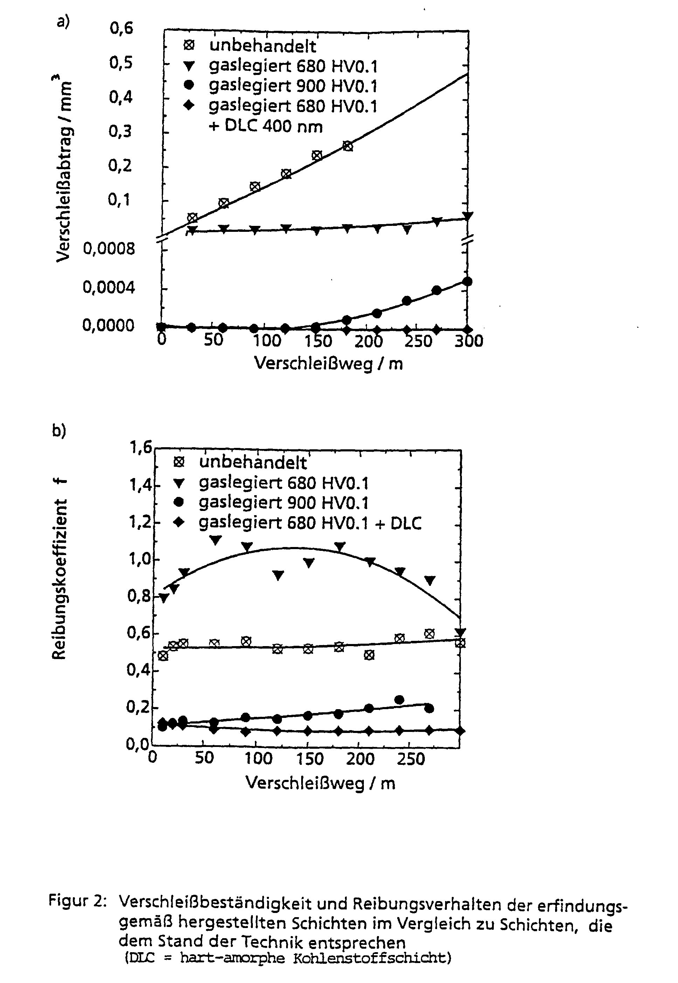

Figur 2: Verschleißbeständigkeit und Reibungsverhalten der erfindungsgemäß hergestellten Schichten im Vergleich zu Schichten, die
dem Stand der Technik entsprechen
(DLC = hart-amorphe Kohlenstoffschicht)